# EUROPEAN PATENT SPECIFICATION

(11) **EP 2 896 420 B1**
(45) Date of publication and mention of the grant of the patent: **27.05.2020**
(21) Application number: 14196904.8
(22) Date of filing: 09.12.2014
(51) Int. Cl.: A61M 5/30, A61M 5/48, A61M 5/20

(54) **Apparatus for needleless transdermal inoculation of medicaments**
Vorrichtung zur nadellosen transdermalen Verabreichung von Arzneimitteln
Appareil pour l'inoculation transdermique de médicaments sans aiguille

(30) Priority: 11.12.2013 IT MI20132067
(43) Date of publication of application: 22.07.2015
(73) Proprietor: Lorini, Dante, 26013 Crema (CR) (IT)
(72) Inventor: Lorini, Dante, 26013 Crema (CR) (IT)
(74) Representative: Raimondi, Margherita

(56) References cited:
- FR-A1- 2 629 706
- US-A- 2 921 582
- US-A- 3 057 349
- US-A- 4 103 684
- US-A- 4 396 384
- US-A1- 2007 167 907

## Description

The present invention relates to an apparatus for transdermal inoculation of medicaments such as vaccines and the like into the muscle of an animal. It is known in the sector of intensive livestock farming of animals such as poultry, pigs and ruminants that there exists the need to administer to said animals medicaments in general, vaccines, antibiotics and the like.

It is also known that such administration is performed by means of intramuscular inoculation in specific given areas of each animal using special syringes equipped with needle and hand-grip and with a plunger which is operated with a pressing force of the operator's thumb against the opposing action of a spring which, once injection has been performed, causes the plunger to return to the initial position for inoculation of a fresh dose of a medicament.

By way of an alternative CA 2 822 908 A1 and WO2012/048268 A2 disclose administration devices which are designed to introduce the medicament without the use of needles, but by using the discharge thrust of the fluid from a chamber which is placed under pressure by means of a quantity of gas contained inside a pressurised cartridge or by the action of a plunger.

In the first case the actual discharge thrust of the gas and therefore the actual effectiveness and repeatability of the inoculation are highly unpredictable, while in the second case the apparatus is very complex and costly owing to the introduction of complicated means for controlling the thrusting force to be applied to the medicament for transdermal inoculation thereof, with the practical difficulty of supplying the inert gas at a high pressure, as well as the high cost of this service, considering also that livestock farms in question are normally located in areas far from urban centres and that in many countries of the world this facility is practically non-existent.

US 3 057 349 describes an inoculation gun in which firing is performed by a spring prestressed by a hydraulic fluid under pressure supplied to a metering chamber, the fluid keeping the spring compressed until the chamber is rapidly emptied of the fluid, allowing firing.

This results in the need to provide two triggers with respective control valves and associated ducts respectively for supplying the fluid under pressure and for fast discharging thereof with the consequent firing and injecting action.

A further example of an injection apparatus according to the prior art is described in FR 2 629 706. The apparatus is formed by a cylindrical container consisting of two parts which can be separated so as to allow insertion of the phial containing the medicament to be injected.

This embodiment gives rise to numerous complications resulting from the need to form an internal space for housing the phial, with the result that the springs for rearming the device must be arranged in opposite positions with respect to the phial itself. In addition, operation of the apparatus is based, on the one hand, on a firing action caused by the feeding of compressed air supplied to the front end of the apparatus and acting in the same direction as that of injection of the medicament and, on the other hand, on a mechanical arming action performed by the springs arranged along the containing cylinder.

Both the known apparatus have a highly complex structure formed by a large number of parts, as well as large dimensions and complex and costly auxiliary operating components.

The technical problem which is posed, therefore, is that of providing an apparatus which provides a solution to the problems of the prior art mentioned above and which in particular is designed to allow the transdermal inoculation, to the correct depth, of medicaments or the like, including also large quantities of vaccine also for large animals.

In connection with this problem it is also required that this apparatus should have small dimensions and be easy and inexpensive to produce and assemble by means of standard apparatus which can be easily used and maintained.

These results are obtained according to the present invention by an apparatus for transdermal inoculation of medicaments according to the features of Claim 1. Advantageous embodiments of the invention are set out in the dependent claims.

Further details may be obtained from the following description of a non-limiting example of embodiment of the subject of the present invention, provided with reference to the accompanying drawings, in which:
Figure 1: is a schematic exploded view partially cross sectioned of the inoculation apparatus according to the present invention and a hand-grip which can be associated with said apparatus;
Figure 2: is a longitudinal section, along a vertical axial plane, of the apparatus according to Fig. 1 in the assembled condition;
Figure 3: is a longitudinal section, similar to that of Fig. 2, of the apparatus during filling;
Figure 4: is a cross-section, similar to that of

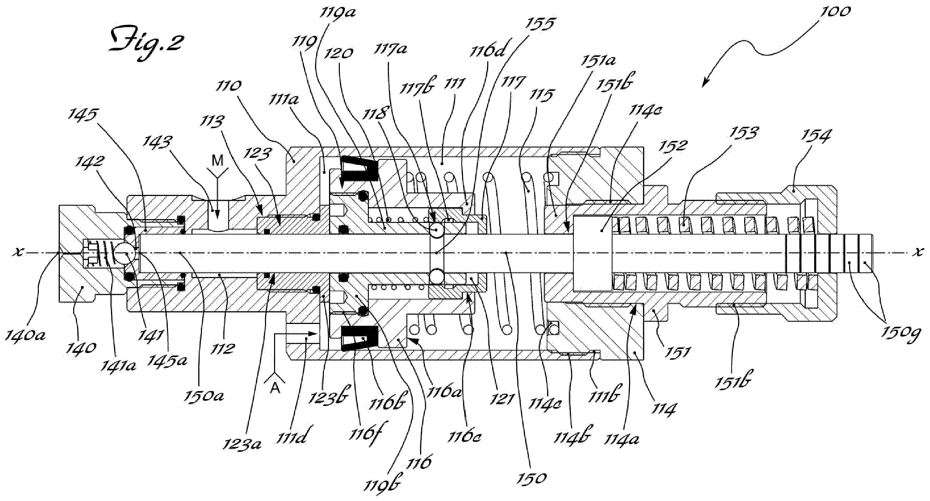

Fig. 2, of the apparatus during dispensing;

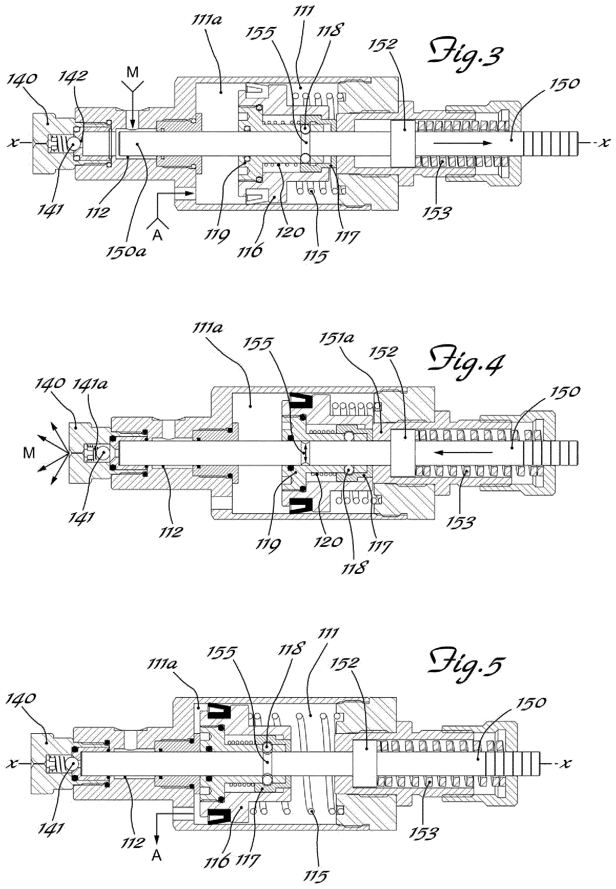

Figure 5: is a cross-section, similar to that of
Fig. 2, of the apparatus during rearming;

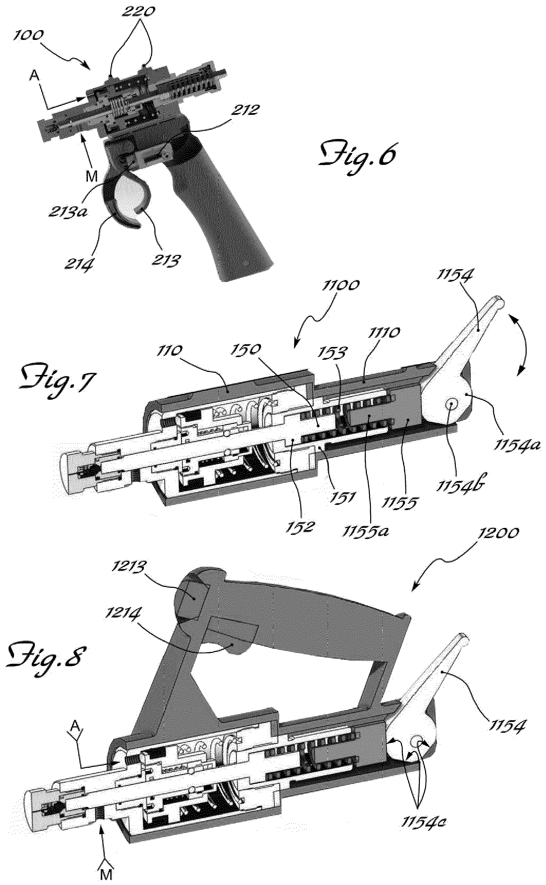

Figure 6: shows the apparatus according to the invention assembled with a hand-grip for gripping and operation by the user;
Figure 7: shows a longitudinally sectioned view of a variation of embodiment of the means for adjusting the inoculation force of the apparatus according to the present invention; and
Figure 8: shows a view, similar to that of Fig. 7, of an apparatus associated with a further embodiment of a hand-grip for the user.

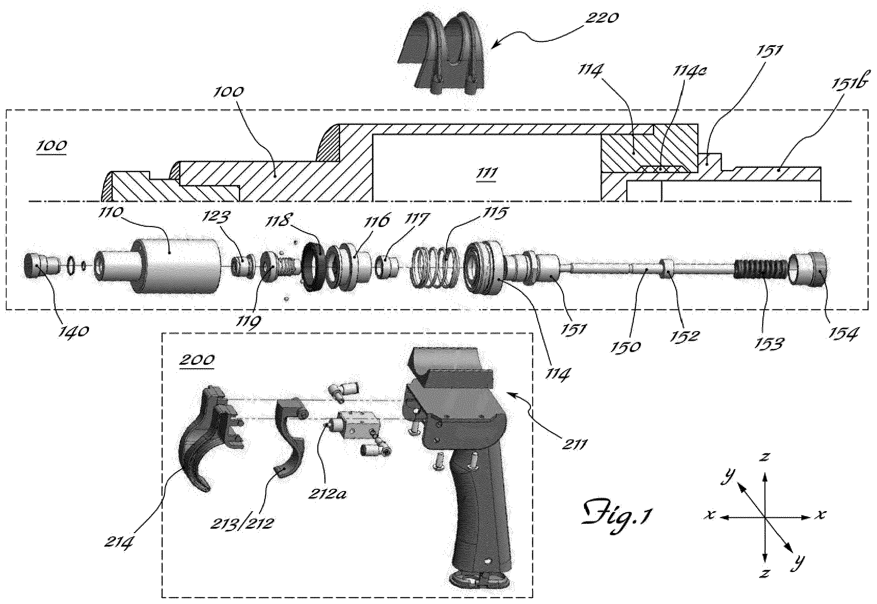

As shown in Figs. 1 and 2 the following axes are assumed solely for the sake of easier description and without a limiting meaning: a set of three axes having, respectively, a longitudinal axial direction X-X, corresponding to the direction of axial extension of the apparatus, transverse/radial direction Y-Y orthogonal to the preceding direction, and vertical direction Z-Z, orthogonal to the plane formed by the other two directions, as well as a front end corresponding to the end for discharging of the medicament and a rear end opposite to said front end.

In addition to this, for the purposes of the present patent, "firing" is understood as meaning the actions which cause the returning of the medicament and "arming" is understood as meaning the actions which cause the resetting of a apparatus to the firing condition.

As shown, an apparatus 100 for transdermal inoculation of medicaments and the like according to the invention comprises :
-) a cylindrical mono-body 110 forming a first, rear, chamber 111 of given diameter and a second, front, chamber 112 for metering the medicament to be injected, having a smaller diameter than that of the first chamber 111, said chambers extending coaxially in the longitudinal direction and being connected together by means of an intermediate chamber 113 having a diameter in between the two said diameters; the cylindrical body has an inlet 111d, which only by way of example is shown as being axial, for supplying to the first chamber 111 compressed air acting in a direction opposite to the direction of inoculation and firing.
-) the rear chamber 111 is delimited at the rear by a first, rear, closing member 114 of the cylindrical mono-body 110, which has a coaxial through-hole 114a and which is mounted on the body 110 for example by means of a screw 114b/female thread 111b connection;
the first chamber 111 is furthermore delimited at the front by a second front closing member 123 in turn provided with a through-hole 123a and an annular head 123b able to enter into bearing contact with the front end surface of the chamber 111; as shown, the diameter of the annular head 123b of the second closing member 123 is smaller than the inner diameter of the chamber, so as to leave an annular volume 111a of the rear chamber 111 free.

On its surface facing the inside of the chamber 111, the first closing member 114 has a seat 114c for receiving the end of a spring 115, the other end of which acts against a corresponding outer bearing surface 116a of a hollow piston 116, in turn arranged coaxially and axially slidably inside the rear chamber 111; the other surface of the piston also has, mounted thereon, an annular seal 116b, preferably of the lip type, for ensuring a relative sealing action for the air A supplied to the chamber 111 through the associated inlet 111d.
-) inside the hollow piston 116 there is, coaxially arranged, a hollow sleeve 117 for carrying balls 118, which has an axially rear part with an outer diameter corresponding to the inner diameter of an open hole 116a in the rear end surface 116d of the piston 116 and a front part with an outer diameter corresponding to the inner diameter of the piston 116; internally the sleeve 117 has an annular projection 117a and an annular seat 117b which are arranged axially in succession from the front end to the rear end of the apparatus and are respectively designed to interfere with and partially contain a plurality of balls 118 designed to interact in the transverse/radial direction with a plunger 150 as will emerge more clearly below;
-) the sleeve 117 is kept axially in bearing contact against the inner rear end of the hollow piston 116 by a substantially T-shaped thruster 119 with a shank 119a having: an outer diameter corresponding to the inner diameter of the hollow sleeve 117, inside which it penetrates partially in the axial direction, and a head 119b with an outer diameter such as to make bearing contact inside a corresponding seat 116f of the base of said piston 116; a coaxial thrust spring 120 substantially concentric with the spring 115 acting against the piston 116 being arranged between the head 119b of the thruster and the front base of the sleeve 117; as shown, once assembly has been completed, a free space 121 remains between the free end of the shank of the T inside the recess and the rear end surface of the sleeve 117.

On the free front end of the said second chamber 112 there is mounted a nozzle 140 which is provided with an associated outlet hole 140a and has, arranged inside it, a ball 141 which is pushed by a spring 141a towards a rear duct 142 connecting the nozzle to the front chamber 112 so that the duct is normally closed.

An adapter 145 with coaxial inner seat 143, inside which the front end of a plunger 150 described below may penetrate, is arranged between the nozzle 140 and the front end of the cylindrical body inside which the front chamber 112 is formed.

A duct 143, which is radial in the example, for supplying the medicament M to be inoculated also leads into the front chamber 112.

Coaxially with the cylindrical body 110 and inside it, a firing plunger 150 is mounted thereon, said plunger being assembled at the rear inside a nipple 151 which is screwed onto the female thread 114c of the through-hole of the member 114 for closing the first chamber 111; in greater detail, the rear part of the plunger 150 has an annular projection 152 with an outer diameter substantially corresponding to the inner diameter of the nipple 151 so as to form an end-of-travel element making contact against the front wall 151a of the nipple provided with a through-hole 151b having an outer diameter corresponding to the outer diameter of the plunger 150; the annular projection 152 of the plunger 150 forms a support base for one end of a firing spring 153, the other end of which reacts against a member 154 for closing the rear of the nipple 151. Preferably, said closing member consists of a ring 154 for adjusting the compression of the spring, which is screwed onto the second thread 151b of said nipple; said ring having a rear hole for allowing the end of the plunger 150 to protrude outwards.

In a zone thereof axially corresponding to the axial position of the sleeve 117 carrying the balls 118, the plunger has an annular groove 155 designed to cooperate with the said balls 118 so as to cause coupling/release of the plunger with/from the piston 116.

In the rest condition the front end 150a of the plunger 150 bears axially against the duct 142 of the nozzle 140.

With reference to this configuration the operating principle of the apparatus is now explained:
-) in the rest and cycle start conditions (Fig. 2) the three springs 115,120,153 all act with a thrusting force, so as to keep, respectively:
   the piston 116 in the end-of-travel position towards the front end of the chamber 111 so that the thruster 119 bears against the annular head 13b of the front closing member 123,
   the sleeve 117 in bearing contact against the rear surface of the piston 116 so that its rear part protrudes partially from the said piston,
   the plunger 150 pushed forward with the end-of-travel projection 152 against the front end wall 151a of the nipple 151;
   in this way the annular seat 155 of the plunger 150 is arranged axially aligned with the balls 118 kept radially pushed towards the longitudinal axis X-X **by** the projection 117a of the sleeve 117: the plunger 150 is therefore rigidly locked to the piston 116;
-) by supplying air A (Fig. 3) to the chamber 111 to act in a direction opposite to the firing direction and with a pressure such as to overcome the thrust of the springs 115 and 153, the piston 116 and the plunger 150 are simultaneously displaced towards the rear of the chamber 111 until the sleeve 117 makes bearing contact axially with the nipple 151 of the rear closing member 114; retraction of the plunger 150 also creates a vacuum inside the front chamber 112 which causes the suction of the predefined quantity of medicament M from a respective storage container (not shown);
-) continuing with supplying of the air A (Fig. 4), the piston continues its axial travel until it comes into contact against the closing member 114, causing simultaneously:
   retraction, towards the front, of the sleeve 117 which consequently brings its annular seat 117b into an axial position corresponding to that of the balls 118 which are thus allowed to come out radially from the annular seat 155 of the plunger 150 which is now disengaged from the condition locked together with the piston 116;
   -) the plunger 150, freed from engagement with the piston 116, is pushed violently forwards (fig. 5) by the thrust of the previously compressed spring 153, causing:
      the medicament M to be discharged through the nozzle 140,
      the return to the initial position (Fig. 2) of the apparatus, with rearming of the plunger which is ready for a second inoculation cycle.

Correct designing of the dimensions of the various component parts and in particular the volumes of the first and second chambers and the reaction force of the three springs will also allow the aspiration/injection of a precise and predefined amount of medicament M; in addition, adjustment, by means of the ring nut 154, of the force of the spring 153 acting on the plunger 150 also allows adjustment of the penetration force and the injection depth of the medicament depending on the hardness of the hide and the size of the animal. According to a preferred embodiment the rear free end of the plunger 150 has annular notches 150g, preferably marked with values of a numerical scale which correspond to different inoculation pressure values related to the different ages and type of animal and determined a priori on the basis of test measurements and which provide the user with a means for intuitive calibration of the thrusting force of the plunger.

It is therefore clear how the apparatus according to the invention is easy and inexpensive to implement, manage, use and maintain, also by personnel who are not particularly skilled, and can be supplied with a fluid, such as compressed air, obtainable at a low cost by means of an ordinary compressor which can be found and installed in any location and at a negligble cost, without depending on any external service.

In addition, in the apparatus according to the invention, the inoculating action is determined by the force imparted by the spring acting on the plunger, while subsequent rearming is performed by means of the supply fluid (compressed air), therefore eliminating the manual effort on the part of the operator with a consequent reduction in the fatigue and improved performance.

Moreover, owing to the particular concentric arrangement of the cylindrical mono-body, the piston with associated arming springs and the radial ball carrier sleeve, the longitudinal dimensions of the apparatus may be kept small, thus resulting in better balance and therefore precision of the apparatus and a reduction in the fatigue of the operator, this fatigue being one of the main causes of imprecise or unsuccessful inoculation. Moreover, with the apparatus according to the invention, use of the inoculation needle is eliminated also for the administration of large quantities of medicament and this results in the further advantages comprising:
- the substantial elimination of the risk of transmission of infections between animals, since, when operating in large numbers, it is not possible to replace every time the needle following use with conventional methods (iatrogenic transmission);
- guarantee of the sterility of the drug or vaccine which is injected;
- a significant reduction in the traumatic effect caused by the introduction of the needle into the body of the animal, with frequent haemorrhages and in particular bacterial infections attributable to dirty or blunt needles (abscesses which are also responsible for a significant economic loss since they result in devaluation of the carcass in terms of product quality);
- elimination of the risk of broken needles remaining in the carcasses, together with the extremely serious consequences for the consumer in the light of the increasingly rigorous food safety regulations; it should be remembered that all the major slaughterhouses must be equipped with metal detectors in order to eliminate this risk.
- reduction of the stress affecting the animal with obvious benefits for the same;
- elimination of the risk of injury for operators owing to accidental injections, in view also of the high production demands which are characteristic of intensive livestock rearing;
- major reduction in the muscular force required of the operator, this being replaced by the mechanical work performed by the compressed air.

As shown in Fig. 6 and Fig. 1, a further embodiment of the apparatus according to the invention is envisaged and comprises an anatomical hand-grip 200 composed of:
- a butt 210 with a top head-piece 211 which houses internally a valve 212 for supplying the air A; the valve 212 is opened by a trigger 213 protected by a guard 214 which acts on a spindle 213a which opens the duct supplying the compressed air from a corresponding storage container/compressor (not shown); the apparatus 100 is also fixed to the head-piece 211 by means of a bridge-piece 220 (Fig. 1) which can be screwed in the vertical direction Z-Z onto said head-piece 211.

As shown in Fig. 7, it is envisaged that in a further embodiment the nipple 151 is coaxially inserted inside an axial extension 1111 of the body 111 and that the axial length of the rear part of the plunger 150 is reduced so as to occupy only a part of the inside of said nipple, so that the other, rear, part may contain the shank 1155a of a thruster 1155, the head 1155b of which external to the nipple 151 forms the bearing surface for the rear end of the spring 153.

The presser 1155 is displaced by the end 1154a of a lever 1154 rotating about a transverse pin 1154b; conveniently the end 1154a of the lever 1154 acting on the presser 1155 is formed as a cam with a plurality of flat surfaces 1154c which are arranged at different inclinations so as to determine different thrusting forces of the presser 155 and therefore different compressions of the spring 153 acting during firing on the plunger 150 with consequent different forces for injection of medicament.

Fig. 8 shows a further embodiment of the hand-grip 1200 which can be associated with the inoculation apparatus 1100 according to Fig. 7; this embodiment has a firing trigger 1213 and a safety locking element 1214 which, only when pressed, releases the supply of air A to the apparatus.

In these configurations the apparatus thus becomes a convenient, light and functional transdermal inoculation gun designed to ensure a high inoculation performance.

Although described in connection with the use for animals, if designed with the appropriate dimensions the apparatus may also be used for administration operations carried out on humans. Although described in connection with a number of embodiments and a number of preferred examples of embodiment of the invention, it is understood that the scope of protection of the present patent is determined solely by the claims below.

## Claims

1. Apparatus for transdermal inoculation of medicaments or the like, extending in a longitudinal axial direction (X-X) between a front end for discharging the medicament in a firing direction and a rear end opposite to the front end, comprising:
• a cylindrical mono-body (110) forming
- a rear chamber (111) of given diameter with a front end surface provided with a front inlet (111d) for air (A) supplied under pressure to act in a direction opposite to the firing direction;
- a front chamber (112) with a diameter smaller than that of the rear chamber (111) and provided with an associated inlet (143) for the medicament (M), said chambers extending coaxially in the longitudinal direction (X-X) and being connected together by means of an intermediate chamber (113) having a diameter in between the diameter of the other two chambers (111;112);
- a first rear closing member (114) for closing the cylindrical mono-body (110), which delimits at the rear the rear chamber (111) and has a coaxial through-hole (114a) with female thread (114c);
- a second front closing member (123), which delimits at the front the rear chamber (111) and has an annular head (123b) able to enter into bearing contact with the front end surface of the rear chamber (111) and which has a through-hole (123a) passing through it;
• a**n arming** hollow piston (116) coaxially arranged in a sealed manner and axially slidably inside the rear chamber (111), the piston (116) comprising:
- an outer bearing surface (116a),
- a rear end surface (116d),
- a hole (116c) formed in the rear end surface (116d),
- a base with a seat (116f);
• a **first** spring (115) arranged between the outer bearing surface (116a) of the piston (116) and the first rear closing member (114)
• a hollow sleeve (117) which is coaxially inserted inside the piston (116) and which has
- an axially rear part with an outer diameter corresponding to the inner diameter of the hole (116c) formed in the rear end surface (116d) of the piston (116);
- a front part with an outer diameter corresponding to the inner diameter of the piston (116);
- internally the sleeve (117) has an annular projection (117a) for interfering with a plurality of balls (118) and an annular seat (117b) for partially containing the plurality of balls (118), which are arranged axially one after another from the front end towards the rear end of the apparatus;
• a substantially T-shaped thruster (119) with:
- a shank (119a) having an outer diameter corresponding to the inner diameter of the hollow sleeve (117) inside which it penetrates partially in the axial direction and
- a head (119b) with an outer diameter such as to make bearing contact inside the corresponding seat (116f) of the base of the piston (116);
• a thrust spring (120) arranged between the head (119b) of the thruster (119) and the front part of the sleeve (117) and concentrically with respect to the spring (115) acting on the arming piston;
• a nozzle (140) which is provided with an associated outlet hole (140a) and has, arranged inside it, a ball (141) which is pushed by a spring (141a) towards a rear duct (142) connecting the nozzle to the front chamber (112) so that the duct is normally closed;
• a firing plunger (150) coaxially contained inside the cylindrical body (110) and provided with a rear part which has an annular projection (152) of given outer diameter;
• a nipple (151) screwed onto the female thread (114c) of the through-hole of the closing member (114), the nipple having
- an inner diameter substantially corresponding to the outer diameter of the annular projection (152) of the rear part of the plunger (150);
- a front wall (151a) having a through-hole (151b) with an inner diameter corresponding to the outer diameter of the plunger (150);
- a rear closing member (154;1154);
• a firing spring (153) arranged between the **rear** closing member (154;1154) of the nipple and the annular projection (152) of the firing plunger (150) acting with a thrust thereon;
wherein the firing plunger (150) is provided with an annular recess (155) suitable for cooperating with the said plurality of balls (118) so as to cause coupling/release of the plunger (150) with/from the piston (116), so that in a rest position of the apparatus, the plunger (150) is pushed forward with the end-of-travel annular projection (152) against the front end wall (151a)of the nipple (151), the annular recess (155)of the plunger (150) being arranged axially aligned with the plurality of balls (118)that are kept radially pushed towards the longitudinal axis (X-X)by the annular projection (117a) of the sleeve (117), such that the plunger (150) is rigidly coupled with the piston (116); and
wherein by supplying air A to the rear chamber (111) via the inlet with a pressure such as to overcome the thrust of the first (115) and firing (153) springs, the piston (116) and the plunger (150) are simultaneously displaced from the rest condition towards the rear of the rear chamber (111) until the sleeve (117) makes bearing contact axially with the nipple (151) of the rear closing member (114), retraction of the plunger (150) creating a vacuum inside the front chamber (112) which causes suction of a predefined quantity of medicament M;
and wherein, continuing with supplying of the air (A), the plunger (150) becomes freed from engagement with the piston (116), and is thus pushed violently forwards by the thrust of the previously compressed firing spring (153), causing the medicament (M) to be discharged through the nozzle (140).

2. Apparatus according to Claim 1, **characterized in that** said member closing the rear end of the nipple (151) is formed by a ring nut (154) screwed onto a rear thread (151b) of the said nipple.

3. Apparatus according to Claim 2, **characterized in that** said ring nut has a rear hole for allowing the rear end of the plunger (150) to protrude externally.

4. Apparatus according to Claim 3, **characterized in that** the rear end of the plunger (150) has annular notches (150g) marked with values of a numerical scale corresponding to different inoculation pressure values.

5. Apparatus according to Claim 1, **characterized in that** the body (111) has an axial extension (1111) inside which the nipple (151) is coaxially inserted and that the axial length of the rear part of the plunger (150) is sufficiently small to occupy only an axial part of the inside of the said nipple.

6. Apparatus according to Claim 5, **characterized in that** it comprises a presser (1155) which has a shank (1155a) contained inside the inner rear part of the nipple (151) and the head (1155b) of which is situated outside the nipple (151) and forms the bearing surface for the rear end of the spring (153).

7. Apparatus according to Claim 6, **characterized in that** it comprises means for adjusting the compression of the firing spring (153), comprising a lever (1154) which is rotatable about a transverse pin (1154b) and the end (1154a) of which acting on the presser (1155) is shaped in the manner of a cam with a plurality of flat surfaces (1154c) arranged at different inclinations.

8. Apparatus according to any one of the preceding claims, **characterized in that** it comprises a hand-grip (200;1200) with associated valve (212) for supplying the compressed air (A), an operating trigger (213;1213) and safety element (214;1214) for preventing accidental firing.

## Patentansprüche

1. Vorrichtung zur transdermalen Verabreichung von Medikamenten oder dergleichen, die sich in Längsrichtung (X-X) zwischen einem vorderen Ende zum Abgeben des Medikaments in Ausstoßrichtung und einem hinteren Ende gegenüber dem vorderen Ende erstreckt und Folgendes umfasst:
• einen zylindrischen Mono-Körper (110) ausbildend
- eine hintere Kammer (111) mit gegebenem Durchmesser mit einer vorderen Endfläche, die mit einem vorderen Einlass (111d) für Luft (A) versehen ist, die unter Druck zugeführt wird, um in einer Richtung entgegengesetzt zur Ausstoßrichtung zu wirken;
- eine vordere Kammer (112) mit einem Durchmesser, der kleiner als der der hinteren Kammer (111) ist und mit einem zugeordneten Einlass (143) für das Medikament (M) versehen ist, wobei sich die Kammern koaxial in Längsrichtung (X-X) erstrecken und mittels einer Zwischenkammer (113) mit einem Durchmesser zwischen dem Durchmesser der anderen beiden Kammern (111; 112) miteinander verbunden sind;
- ein erstes hinteres Schließelement (114) zum Schließen des zylindrischen Mono-Körpers (110), das die hintere Kammer (111) hinten begrenzt und eine koaxiale Durchgangsbohrung (114a) mit Innengewinde (114c) aufweist;
- ein zweites vorderes Schließelement (123), das die hintere Kammer (111) vorne begrenzt und einen ringförmigen Kopf (123b) aufweist, der in Lagerkontakt mit der vorderen Endfläche der hinteren Kammer (111) treten kann und das ein durch dieses hindurchgehendes Durchgangsloch (123a) hat;
• einen hohlen Scharfschaltkolben (116), der koaxial abgedichtet und axial verschiebbar in der hinteren Kammer (111) angeordnet ist, wobei der Kolben (116) umfasst:
- eine äußere Lagerfläche (116a),
- eine hintere Endfläche (116d),
- ein Loch (116c), das in der hinteren Endfläche (116d) ausgebildet ist,
- eine Basis mit einem Sitz (116f);
• eine erste Feder (115), die zwischen der äußeren Lagerfläche (116a) des Kolbens (116) und dem ersten hinteren Schließelement (114) angeordnet ist;
• eine hohle Hülse (117), die koaxial in den Kolben (116) eingesetzt ist und die umfasst:
- einen axial hinteren Teil mit einem Außendurchmesser, der dem Innendurchmesser des Lochs (116c) entspricht, das in der hinteren Endfläche (116d) des Kolbens (116) ausgebildet ist;
- ein vorderes Teil mit einem Außendurchmesser, der dem Innendurchmesser des Kolbens (116) entspricht;
- die Hülse (117) hat innen einen ringförmigen Vorsprung (117a) zum Zusammenwirken mit einer Vielzahl von Kugeln (118) und einen ringförmigen Sitz (117b) zur teilweisen Aufnahme der Vielzahl von Kugeln (118), die axial hintereinander von dem vorderen Ende bis zu dem hinteren Ende der Vorrichtung angeordnet sind;
• ein im wesentlichen T-förmiges Schubstück (119) mit:
- einem Schaft (119a) mit einem Außendurchmesser, der dem Innendurchmesser der hohlen Hülse (117) entspricht, in die es teilweise in axialer Richtung eindringt, und
- einem Kopf (119b) mit einem Außendurchmesser, um einen Lagerkontakt innerhalb des entsprechenden Sitzes (116f) der Basis des Kolbens (116) herzustellen;
• eine Schubfeder (120), die zwischen dem Kopf (119b) des Schubstücks (119) und dem vorderen Teil der Hülse (117) und konzentrisch zu der auf den Scharfschaltkolben wirkenden Feder (115) angeordnet ist;
• eine Düse (140), die mit einem zugehörigen Auslassloch (140a) versehen ist und in der eine Kugel (141) angeordnet ist, die von einer Feder (141a) in Richtung eines hinteren Kanals (142) gedrückt wird, der die Düse mit der vordere Kammer (112) verbindet, so dass der Kanal normalerweise geschlossen ist;
• einen Ausstoßstößel (150), der koaxial in dem zylindrischen Körper (110) enthalten ist und mit einem hinteren Teil versehen ist, der einen ringförmigen Vorsprung (152) mit gegebenem Außendurchmesser aufweist;
• einen Nippel (151), der auf das Innengewinde (114c) des Durchgangslochs des Schließelements (114) aufgeschraubt ist, wobei der Nippel
- einen Innendurchmesser aufweist, der im Wesentlichen dem Außendurchmesser des ringförmigen Vorsprungs (152) des rückseitigen Teils des Stößels (150) entspricht;
- eine Vorderwand (151a) mit einem Durchgangsloch (151b) mit einem Innendurchmesser, der dem Außendurchmesser des Stößels (150) entspricht;
- ein hinteres Schließelement (154; 1154);
• eine Ausstoßfeder (153), die zwischen dem hinteren Schließelement (154; 1154) des Nippels und dem ringförmigen Vorsprung (152) des Ausstoßstößels (150) angeordnet ist und mit einer Schubkraft darauf wirkt;
wobei der Ausstoßstößel (150) mit einer ringförmigen Aussparung (155) versehen ist, die geeignet ist, mit der Vielzahl von Kugeln (118) zusammenzuwirken, um das Koppeln/Freigeben des Stößels (150) mit/von dem Kolben (116) zu bewirken, so dass in einer Ruheposition der Vorrichtung der Stößel (150) mit dem bewegungsbegrenzenden ringförmigen Vorsprung (152) gegen die Vorderwand (151a) des Nippels (151) nach vorne gedrückt wird, wobei die ringförmige Aussparung (155) des Stößels (150) axial ausgerichtet mit der Vielzahl von Kugeln (118) angeordnet ist, die durch den ringförmigen Vorsprung (117a) der Hülse (117) radial in Richtung der Längsachse (X-X) gedrückt gehalten werden, so dass der Stößel (150) starr mit dem Kolben (116) gekoppelt ist;
und wobei durch die Zufuhr von Luft (A) in die hintere Kammer (111) über den Einlass mit einem Druck, der die Schubkraft der ersten Feder (115) und der Ausstoßfeder (153) überwindet, der Kolben (116) und der Stößel (150) gleichzeitig aus dem Ruhezustand in Richtung der Rückseite der hinteren Kammer (111) verschoben werden, bis die Hülse (117) in axialen Lagerkontakt mit dem Nippel (151) des hinteren Verschlusselements (114) kommt, wobei durch das Zurückziehen des Stößels (150) ein Vakuum in der vorderen Kammer (112) erzeugt wird, das das Ansaugen einer vordefinierten Menge an Medikament (M) bewirkt;
und wobei der Stößel (150) bei Fortsetzung der Luftzufuhr (A) aus dem Eingriff mit dem Kolben (116) befreit wird und somit durch die Schubkraft der zuvor zusammengedrückten Ausstoßfeder (153) heftig nach vorne gedrückt wird, wodurch das Medikament (M) durch die Düse (140) abgegeben wird.

2. Vorrichtung nach Anspruch 1,
**dadurch gekennzeichnet,**
**dass** das das hintere Ende des Nippels (151) verschließende Element durch eine Ringmutter (154) gebildet ist, die auf ein hinteres Gewinde (151b) des Nippels aufgeschraubt ist.

3. Vorrichtung nach Anspruch 2,
**dadurch gekennzeichnet,**
**dass** die Ringmutter ein hinteres Loch aufweist, damit das hintere Ende des Bolzens (150) nach außen vorstehen kann.

4. Vorrichtung nach Anspruch 3,
**dadurch gekennzeichnet,**
**dass** das hintere Ende des Stößels (150) ringförmige Kerben (150g) aufweist, die mit Werten einer numerischen Skala gekennzeichnet sind, die verschiedenen Verabreichungsdruckwerten entsprechen.

5. Vorrichtung nach Anspruch 1,
**dadurch gekennzeichnet,**
**dass** der Körper (111) eine axiale Verlängerung (1111) aufweist, in die der Nippel (151) koaxial eingesetzt ist und dass die axiale Länge des hinteren Teils des Stößels (150) ausreichend klein ist, um nur einen axialen Teil der Innenseite des Nippels aufzunehmen.

6. Vorrichtung nach Anspruch 5,
**dadurch gekennzeichnet,**
**dass** sie einen Drücker (1155) umfasst, der einen Schaft (1155a) aufweist, der im inneren hinteren Teil des Nippels (151) enthalten ist und dessen Kopf (1155b) außerhalb des Nippels (151) liegt und die Auflagefläche für das hintere Ende der Feder (153) bildet.

7. Vorrichtung nach Anspruch 6,
**dadurch gekennzeichnet,**
**dass** sie Mittel zum Einstellen der Kompression der Ausstoßfeder (153) umfasst, die einen Hebel (1154) umfassen, der um einen Querbolzen (1154b) drehbar ist und dessen auf den Drücker (1155) einwirkendes Ende (1154a) die Form eines Nockens mit mehreren ebenen Flächen (1154c) aufweist, die in unterschiedlichen Neigungen angeordnet sind.

8. Vorrichtung nach einem der vorhergehenden Ansprüche,
**dadurch gekennzeichnet,**
**dass** sie einen Handgriff (200; 1200) mit zugeordnetem Ventil (212) zum Zuführen der Druckluft (A), einen Betätigungshebel (213; 1213) und ein Sicherheitselement umfasst (214; 1214) zum Verhindern eines versehentlichen Ausstoßes.

## Revendications

1. Appareil d'inoculation transdermique de médicaments, ou analogues, s'étendant dans une direction axiale longitudinale (X-X) entre une extrémité avant destinée à décharger le médicament dans un sens de tir et une extrémité arrière située à l'opposé de l'extrémité avant, comprenant :
un mono-corps cylindrique (110) formant
- une chambre arrière (111) de diamètre donné dotée d'une surface d'extrémité avant pourvue d'une entrée avant (111d) d'air (A) alimenté sous pression pour agir dans un sens contraire au sens de tir ;
- une chambre avant (112) de diamètre plus petit que celui de la chambre arrière (111) et pourvue d'une entrée associée (143) destinée au médicament (M),
lesdites chambres s'étendant coaxialement dans la direction longitudinale (X-X) et reliées l'une à l'autre au moyen d'une chambre intermédiaire (113) ayant un diamètre compris entre les diamètres des deux autres chambres (111 ; 112) ;
- un premier élément de fermeture arrière (114) destiné à fermer le mono-corps cylindrique (110), qui établit une délimitation au niveau de l'arrière de la chambre arrière (111) et qui comporte un trou traversant coaxial (114a) doté d'un filetage femelle (114c) ;
- un second élément de fermeture avant (123), qui établit une délimitation au niveau de l'avant de la chambre arrière (111) et qui comporte une tête annulaire (123b) pouvant entrer en contact d'appui avec la surface d'extrémité avant de la chambre arrière (111) et qui comporte un trou traversant (123a) le traversant ;
un piston creux d'armement (116) disposé coaxialement de manière scellée et pouvant coulisser axialement à l'intérieur de la chambre arrière (111), le piston (116) comprenant :
- une surface d'appui extérieure (116a),
- une surface d'extrémité arrière (116d),
- un trou (116c) formé dans la surface d'extrémité arrière (116d),
- une base dotée d'un siège (116f) ;
un premier ressort (115) disposé entre la surface d'appui extérieure (116a) du piston (116) et le premier élément de fermeture arrière (114)
un manchon creux (117) qui est introduit coaxialement à l'intérieur du piston (116) et qui comporte
- une partie axialement arrière ayant un diamètre extérieur correspondant au diamètre intérieur du trou (116c) formé dans la surface d'extrémité arrière (116d) du piston (116) ;
- une partie avant ayant un diamètre extérieur correspondant au diamètre intérieur du piston (116) ;
- le manchon (117) comportant, intérieurement, une saillie annulaire (117a) destinée à interagir avec une pluralité de billes (118) et un siège annulaire (117b) destiné à contenir partiellement la pluralité de billes (118), qui sont disposés axialement l'un après l'autre de l'extrémité avant vers l'extrémité arrière de l'appareil ;
un propulseur sensiblement en forme de T (119), comprenant :
- une tige (119a) ayant un diamètre extérieur correspondant au diamètre intérieur du manchon creux (117) à l'intérieur duquel elle pénètre partiellement dans la direction axiale, et
- une tête (119b) ayant un diamètre extérieur permettant d'établir un contact d'appui à l'intérieur du siège correspondant (116f) de la base du piston (116) ;
un ressort de poussée (120) disposé entre la tête (119b) du propulseur (119) et la partie avant du manchon (117) et de manière concentrique au ressort (115) agissant sur le piston d'armement ;
une buse (140) pourvue d'un trou de sortie associé (140a) et qui comporte, disposée à l'intérieur de ce dernier, une bille (141) poussée par un ressort (141a) en direction d'un conduit arrière (142) reliant la buse et la chambre avant (112) de façon à fermer normalement le conduit ;
un piston de tir (150) contenu coaxialement à l'intérieur du corps cylindrique (110) et pourvu :
d'une partie arrière qui comporte une saillie annulaire (152) de diamètre extérieur donné ;
un raccord fileté (151) vissé sur le filetage femelle (114c) du trou traversant de l'élément de fermeture (114), le raccord fileté ayant
- un diamètre intérieur correspondant sensiblement au diamètre extérieur de la saillie annulaire (152) de la partie arrière du piston de tir (150) ; et comportant
- une paroi avant (151a) comportant un trou traversant (151b) ayant un diamètre intérieur correspondant au diamètre extérieur du piston de tir (150) ;
- un élément de fermeture arrière (154 ; 1154) ;
un ressort de tir (153) disposé entre l'élément de fermeture arrière (154 ; 1154) du raccord fileté et la saillie annulaire (152) du piston de tir (150) agissant par poussée sur ce dernier ;
dans lequel le piston de tir (150) est pourvu d'un évidement annulaire (155) approprié pour coopérer avec la pluralité de billes (118) de façon à provoquer un accouplement/désaccouplement du piston de tir (150) avec le/du piston (116), de sorte que, dans une position de repos de l'appareil, le piston de tir (150) soit poussé vers l'avant, la saillie annulaire (152) se trouvant en fin de course contre la paroi d'extrémité avant (151a) du raccord fileté (151), l'évidement annulaire (155) du piston de tir (150) étant axialement aligné avec la pluralité de billes (118) qui sont maintenues poussées radialement vers l'axe longitudinal (X-X) par la saillie annulaire (117a) du manchon (117), de façon à accoupler rigidement le piston de tir (150) et le piston (116) ; et
dans lequel, en alimentant la chambre arrière (111) en air A sous pression par le biais de l'entrée de façon à surmonter la poussée du premier ressort (115) et du ressort de tir (153), le piston (116) et le piston de tir (150) sont décalés simultanément de l'état de repos en direction de l'arrière de la chambre arrière (111) jusqu'à ce que le manchon (117) établisse un contact d'appui axialement avec le raccord fileté (151) de l'élément de fermeture arrière (114), un retrait du piston de tir (150) créant un vide à l'intérieur de la chambre avant (112) qui provoque une aspiration d'une quantité prédéfinie de médicament M ;
et dans lequel, le fait de continuer l'alimentation en air (A) libère le piston de tir (150) de la coopération avec le piston (116), et le pousse ainsi violemment vers l'avant du fait de la poussée du ressort de tir comprimé au préalable (153), provoquant une décharge du médicament (M) à travers la buse (140).

2. Appareil selon la revendication 1, **caractérisé en ce que** ledit élément fermant l'extrémité arrière du raccord fileté (151) est formé par un écrou à œillet (154) vissé sur un filetage arrière (151b) dudit raccord fileté.

3. Appareil selon la revendication 2, **caractérisé en ce que** ledit écrou à œillet comporte un trou arrière destiné à permettre un dépassement extérieur de l'extrémité arrière du piston de tir (150).

4. Appareil selon la revendication 3, **caractérisé en ce que** l'extrémité arrière du piston de tir (150) comporte des encoches annulaires (150g) marquées de valeurs d'une échelle numérique correspondant à différentes valeurs de pression d'inoculation.

5. Appareil selon la revendication 1, **caractérisé en ce que** le corps (111) comporte un prolongement axial (1111) à l'intérieur duquel est introduit coaxialement le raccord fileté (151) et **en ce que** la longueur axiale de la partie arrière du piston de tir (150) est suffisamment petite pour n'occuper qu'une partie axiale de l'intérieur dudit raccord fileté.

6. Appareil selon la revendication 5, **caractérisé en ce qu'**il comprend un presseur (1155) qui comporte une tige (1155a) contenue à l'intérieur de la partie arrière intérieure du raccord fileté (151) et dont la tête (1155b) est située à l'extérieur du raccord fileté (151) et forme la surface d'appui de l'extrémité arrière du ressort (153).

7. Appareil selon la revendication 6, **caractérisé en ce qu'**il comprend un moyen permettant de régler la compression du ressort de tir (153), comprenant un levier (1154) qui peut pivoter autour d'une broche transversale (1154b) et dont l'extrémité (1154a) qui agit sur le presseur (1155) est formée à la manière d'une came dotée d'une pluralité de surfaces plates (1154c) disposées à des inclinaisons différentes.

8. Appareil selon l'une quelconque des revendications précédentes, **caractérisé en ce qu'**il comprend une poignée (200 ; 1200) dotée d'une soupape associée (212) destinée à l'alimentation en air comprimé (A), une détente de mise en œuvre (213 ; 1213) et un élément de sécurité (214 ; 1214) destiné à assurer une prévention contre un tir accidentel.
